# EUROPÄISCHE PATENTSCHRIFT

(11) **EP 3 796 373 B1**
(45) Veröffentlichungstag und Bekanntmachung des Hinweises auf die Patenterteilung: **28.06.2023**
(21) Anmeldenummer: 19198533.2
(22) Anmeldetag: 20.09.2019
(51) Int. Cl.: H01L 23/00

(54) **PLATINENANORDNUNG EINES IMPLANTIERBAREN MEDIZINISCHEN GERÄTS**
CIRCUIT BOARD ASSEMBLY OF IMPLANTABLE MEDICAL DEVICE
AGENCEMENT DE PLATINE D'UN APPAREIL MÉDICAL IMPLANTABLE

(43) Veröffentlichungstag der Anmeldung: 24.03.2021
(73) Patentinhaber: BIOTRONIK SE & Co. KG, 12359 Berlin (DE)
(72) Erfinder: MEIER, Roman, 83137 Schonstett (DE); QUAHS, Dany, 95111 Rehau (DE); ROMBERG, Jan, 12347 Berlin (DE)
(74) Vertreter: Biotronik Corporate Services SE

(56) Entgegenhaltungen:
- EP-A1- 2 852 970
- EP-A1- 3 428 600
- JP-A- 2006 047 287
- JP-A- 2015 075 422

## Beschreibung

Die Erfindung betrifft eine Platinenanordnung eines implantierbaren medizinischen Geräts, ein implantierbares medizinisches Gerät und ein Verfahren zum Herstellen einer Platinenanordnung eines implantierbaren medizinischen Geräts.

Eine bekannte Platinenanordnung umfasst eine Platine und eine an der Platine angeordnete Sensoreinrichtung, die über eine Klebeschicht mit der Platine verbunden ist.

Eine solche Platinenanordnung ist Bestandteil eines medizinischen Geräts, beispielsweise eines implantierbaren Drucksensors, der in einen Patienten zu implantieren ist, zum Beispiel um den Blutdruck in einem Blutgefäß, insbesondere im Bereich des menschlichen Herzens, intravaskulär oder intrakardial zu überwachen.

Bei einem solchen implantierbaren Drucksensor ist eine Sensoreinrichtung, die beispielsweise eine Druckmembran zum Messen eines Fluiddrucks aufweist, auf einer Platine angeordnet und über die Platine in einem Gehäuse des medizinischen Geräts eingefasst. Die Platine ist hierbei an dem Gehäuse befestigt und trägt weitere elektrische oder elektronische Komponenten, beispielsweise zur Verwirklichung einer Elektronik zum Vorverarbeiten von über die Sensoreinrichtung aufgenommenen Sensorsignalen, die als elektrische Signale den elektrischen oder elektronischen Komponenten zugeführt werden.

In montiertem Zustand ist die Platine mit der daran angeordneten Sensoreinrichtung üblicherweise fest mit einem umgebenden Gehäuse verbunden. Dies führt dazu, dass mechanische Spannungen, die über das Gehäuse in die Platine eingeleitet werden, auch zu einer Belastung an der Sensoreinrichtung führen und unter Umständen Sensorsignale verfälschen können, was es zu vermeiden gilt.

Um eine mechanische Entkopplung der Sensoreinrichtung von der Platine insbesondere entlang einer parallel zur Oberfläche der Platine gerichteten Ebene zu erreichen, ist die Sensoreinrichtung über eine Klebeschicht mit der Platine stoffschlüssig verbunden, wobei für eine wirkungsvolle mechanische Entkopplung die Klebeschicht eine hinreichende Dicke aufweisen sollte, um insbesondere zu verhindern, dass die Sensoreinrichtung gegebenenfalls auf im Rahmen einer toleranzbehafteten Fertigung an der Platine bestehenden Vorsprüngen oder dergleichen aufliegt, was ansonsten mit einer ungewünschten mechanischen Kopplung einhergehen könnte.

Wünschenswert ist hierbei eine Lösung, bei der die Klebeschicht mit hinreichender Prozesssicherheit in definierter Weise in ihrer Schichtdicke eingestellt werden kann.

Aus dem Dokument EP 3 428 600 A1 ist ein Klebstoff zum Verbinden eines Halbleitersensors mit einem Substrat bekannt. Der Klebstoff ist durch einen Silikonkleber gebildet, der sogenannte Spacer in Form von Silikonpartikeln aufweist.

Das Dokument US 2003/0043476 A1 beschreibt eine Verbindung zwischen einem optischen Gerät und einer Trägerplatte.

EP 2 852 970 A1 offenbart ein Verfahren zum Herstellen einer elektronischen Baugruppe. Aufgabe der vorliegenden Erfindung ist es, eine Platinenanordnung, ein implantierbares medizinisches Gerät und ein Verfahren zum Herstellen einer Platinenanordnung zur Verfügung zu stellen, die auf einfache, kostengünstige Weise eine Verbindung einer Sensoreinrichtung mit einer Platine zum Einbau in ein medizinisches Gerät bei vorteilhafter mechanischer Entkopplung und verbesserter Prozesssicherheit ermöglichen.

Es sind eine Platinenanordnung nach Anspruch 1, ein implantierbares medizinisches Gerät nach Anspruch 6 und ein Verfahren nach Anspruch 7 bereitgestellt. Weitere Ausführungsformen sind Gegenstand von abhängigen Ansprüchen.

Gemäß einem ersten Aspekt ist Platinenanordnung eines implantierbaren medizinischen Geräts bereitgestellt. Die Platinenanordnung umfasst eine Platine und eine an der Platine angeordnete Sensoreinrichtung. Die Sensoreinrichtung ist über eine Klebeschicht mit der Platine verbunden. Die Klebeschicht ist aus einer Klebstoffmasse gebildet, in die Glaskugeln eingebettet sind.

Die Verbindung der Sensoreinrichtung, die insbesondere als Transducer zum Wandeln eines Drucksignals in ein elektrisches Signal ausgebildet ist, mit der Platine erfolgt über eine Klebeschicht, die aus einer mit Glaskugeln vermengten Klebstoffmasse hergestellt ist. Die Glaskugeln weisen eine definierte Größe, insbesondere einen Durchmesser in einem definierten Bereich, auf und dienen in der Klebstoffmasse als Abstandshalter, um zu gewährleisten, dass die Klebeschicht eine Mindestdicke nicht unterschreiten kann und somit die Sensoreinrichtung über eine zur mechanischen Entkopplung hinreichend dicke Klebeschicht mit der Platine verbunden ist.

Die Glaskugeln weisen einen Elastizitätsmodul zwischen 40.000 N/mm² und 90.000 N/mm² auf und sind somit vergleichsweise starr und unelastisch ausgebildet, sodass die Glaskugeln unnachgiebig sind und auch bei vergleichsweise großem Druck beim Fügen der Sensoreinrichtung auf der Platine es nicht zu einer Verformung an den Glaskugeln kommen kann.

Die Glaskugeln können eine sphärische Form mit kreisrundem Querschnitt aufweisen. Die Form der Glaskugeln kann aber auch von einer idealen Kugelform abweichen, beispielsweise indem die Glaskugeln im Querschnitt abweichend von einer kreisrunden Form ausgebildet, beispielsweise elliptisch geformt sind.

Die Glaskugeln können beispielsweise einen Durchmesser zwischen 20 µm und 100 µm, beispielsweise zwischen 25 µm und 80 µm, vorzugsweise zwischen 30 µm und 50 µm aufweisen. Dadurch, dass die Glaskugeln einen Durchmesser in einem definierten Bereich aufweisen und sich die Glaskugeln in ihrem Durchmesser somit vorzugsweise nur geringfügig unterscheiden, kann über die Glaskugeln in der Klebeschicht eine günstige, gleichförmige Abstützung der Sensoreinrichtung gegenüber der Platine geschaffen werden, insbesondere entlang einer zur Oberfläche der Platine senkrechten Normalenrichtung, entlang derer die Sensoreinrichtung an die Oberfläche der Platine angesetzt ist.

Der Anteil der Glaskugeln in der Klebeschicht beträgt hierbei zwischen 5 Gew.% und 40 Gew.%, beispielsweise zwischen 8 Gew.% und 20 Gew.%, beispielsweise 10 Gew.%,. Durch einen hinreichend großen Anteil von Glaskugeln in der Klebeschicht kann eine verteilte Abstützung der Sensoreinrichtung gegenüber der Platine mit homogener Verteilung der Glaskugeln in der Klebeschicht und vergleichsweise geringem Abstand zwischen benachbarten Glaskugeln geschaffen werden, sodass eine definierte Zwischenlage zwischen der Sensoreinrichtung und der Platine geschaffen wird, bei stoffschlüssiger (klebender) Verbindung und definierter Schichtdicke.

Die Klebstoffmasse ist vorzugsweise durch einen Klebstoff gebildet, der auch in ausgehärtetem Zustand deutlich weicher als die Glaskugeln ist. Während die Sensoreinrichtung entlang der Normalen zur Platinenfläche somit vergleichsweise starr über die Glaskugeln gegenüber der Platine abgestützt ist, wird eine Entkopplung zwischen der Sensoreinrichtung und der Platine entlang der beiden anderen Raumrichtungen geschaffen, welche parallel zur Platinenfläche sind. Mechanische Spannungen, die insbesondere horizontal entlang der Platine wirken und beispielsweise durch eine Verformung an der Platine bedingt sind, können somit entlang der Ebene der Platine nicht auf die Sensoreinrichtung übertragen werden, sodass die Sensoreinrichtung mechanisch von der Platine entkoppelt ist. Eine Verfälschung von Sensorsignalen, insbesondere ein Driften von Sensorsignalen, infolge von Verspannungen und Verformungen an der Platine ist somit wirkungsvoll verhindert.

Aufgrund der Elastizität der Klebstoffmasse können darüber hinaus auch Verspannungen entlang der Normalenrichtung, beispielsweise infolge eines Verbiegens an der Platine, ausgeglichen werden, sodass solche Verspannungen nicht unmittelbar auf die Sensoreinrichtung übertragen werden können und zumindest stark gedämpft werden.

Die Klebstoffmasse kann beispielsweise durch einen Silikonkleber gebildet sein. Ein solcher Silikonkleber kann eine hohe Kettenbeweglichkeit von Silikonpolymeren aufweisen, die zu einer vergleichsweise großen Elastizität auch der ausgehärteten Klebstoffmasse führt. Solche Silikonkleber können temperaturbeständig und zudem beständig gegen UV-Strahlung sein. Silikonkleber können zudem resistent gegen Feuchtigkeit sein.

Silikonkleber können als 1-Komponenten- oder 2-Komponenten-Klebstoffsysteme verwendet werden. Sowohl 1-Komponenten-Systeme als auch 2-Komponenten-Systeme beruhen auf Polyorgano-, meist Polydimethylsiloxanen und härten durch eine Polykondensation aus.

In einer Ausgestaltung ist die Sensoreinrichtung als Transducer zum Wandeln eines Drucksignals in ein elektrisches Signal ausgebildet. Die Sensoreinrichtung kann beispielsweise eine Druckmembran aufweisen, die mit einem Fluid in Kontakt gebracht werden kann und somit einen Druck in dem Fluid aufnehmen kann. Alternativ kann die Sensoreinrichtung beispielsweise piezoelektrisch zum Wandeln eines Drucksignals in ein korreliertes elektrisches Signal ausgebildet sein.

In einer Ausgestaltung weist ein implantierbares medizinisches Gerät eine Platinenanordnung der vorangehend beschriebenen Art auf. Ein solches implantierbares medizinisches Gerät weist ein Gehäuse auf, in dem die Platinenanordnung eingefasst ist. Die Platinenanordnung ist hierbei üblicherweise fest mit dem Gehäuse verbunden und weist - neben der Sensoreinrichtung - weitere elektrische oder elektronische Komponenten auf, über die eine Signalverarbeitung, insbesondere eine Vorverarbeitung von über die Sensoreinrichtung erhaltenen Sensorsignalen, erfolgen kann.

Die Einbausituation der Platinenanordnung in das implantierbare medizinische Gerät kann als einseitige Einspannung ausgeführt sein (ähnlich eines Sprungbrettes). Alternativ kann die Einbausituation als zweiseitige Einspannung ausgeführt werden, was konstruktiv einfacher ist.

Die Platinenanordnung kann eine oder mehrere FR4-Platine umfassen. Die Platinenanordnung kann eine oder mehrere Platinen auf keramischer Mehrlagen-Basis (LTCC - Low Temperature Cofired Ceramics, dt.: Niedertemperatur-Einbrand-Keramiken) aufweisen. Eine flexible Platine reagiert auf mechanische Spannungen mit Verformung, einer Art Wellung. Im Bereich des starren Transducers resultieren diese Wellen dann wieder in Verspannungen. Daher sind möglichst steife Platinen zu bevorzugen.

Ein solches implantierbares medizinisches Gerät kann beispielsweise als implantierbarer Drucksensor ausgebildet sein, der in ein menschliches Gefäß, insbesondere ein Blutgefäß, implantierbar ist, um den Druck in dem Gefäß zu erfassen, beispielsweise zum Überwachen des (lokalen) Blutdrucks über einen längeren Zeitraum. Ein solches medizinisches Gerät in Form eines implantierbaren Drucksensors kann beispielsweise im Bereich des Herzens, beispielsweise intrakardial, implantiert sein. Der Drucksensor kann mittels einer Leitung mit einer ebenfalls implantierten, aber außerhalb des Herzens angeordneten Steuereinrichtung verbunden sein. Die Steuereinrichtung kann beispielsweise eine Steuerelektronik zur Auswertung von Sensorsignalen umfassen. Die Steuereinrichtung kann zudem beispielsweise Kommunikationskomponenten aufweisen, über die eine Signalübertragung an eine externe Einheit außerhalb des Patienten zum Beispiel mittels Telemetrie vorgenommen werden kann. In einer anderen Ausführungsform kann der Drucksensor selbst Kommunikationskomponenten aufweisen und konfiguriert sein, drahtlos mit einem außerhalb des Patienten befindlichen Gerät zu kommunizieren, z.B. um Daten an das Gerät zu übertragen und/oder Programmierbefehle von dem Gerät zu empfangen. Das Gerät kann beispielsweise ein Smartphone oder ein Tablet sein.

Die Aufgabe wird auch gelöst durch ein Verfahren zum Herstellen einer Platinenanordnung eines implantierbaren medizinischen Geräts, bei dem eine Sensoreinrichtung über eine Klebeschicht mit einer Platine verbunden wird. Die Klebeschicht wird aus einer Klebstoffmasse gebildet, in die Glaskugeln eingemischt sind.

Die vorangehend für die Platinenanordnung und das medizinische Gerät beschriebenen Vorteile und vorteilhaften Ausgestaltungen finden analog auch auf das Verfahren Anwendung, sodass diesbezüglich auf das vorangehend Ausgeführte verwiesen wird.

Bei der Klebstoffmasse kann es sich insbesondere um einen Silikonkleber handeln, der als 1-Komponenten-Klebstoffsystem oder 2-Komponenten-Klebstoffsystem vorliegen kann.

Handelt es sich bei der Klebstoffmasse um ein 2-Komponenten-System, so werden die Komponenten der Klebstoffmasse vorzugsweise zunächst miteinander vermischt, um erst anschließend die Glaskugeln in die Klebstoffmasse einzumischen. Beim Mischen sollten Blasen vermieden werden, um Lufteinschlüsse in der Klebstoffmasse zu verhindern.

Die in die Klebstoffmasse eingemischten Glaskugeln weisen hierbei vorzugsweise einen Durchmesser in einem definierten Bereich, zum Beispiel zwischen 20 µm und 100 µm, beispielsweise zwischen 25 und 80 µm, vorzugsweise zwischen 30 und 50 µm, auf. Solche Glaskugeln können zum Beispiel durch Sieben erhalten werden, indem ein gewünschter Anteil von Glaskugeln mit einem Durchmesser in einem definierten Bereich ausgesondert und ausschließlich zur Vermengung mit der Klebstoffmasse verwendet wird.

Zum Fügen der Sensoreinrichtung mit der Platine wird die Sensoreinrichtung entlang der Normalenrichtung der Platine unter Zwischenlage der mit den Glaskugeln vermengten Klebstoffmasse an die Platine angesetzt. Über die Glaskugeln wird die Sensoreinrichtung beim Fügen derart gegenüber der Platine abgestützt, dass eine Klebeschicht mit definierter Dicke erhalten wird, die insbesondere eine durch die Glaskugeln vorgegebene Mindestdicke nicht unterschreitet.

Weil die Sensoreinrichtung über die in der Klebeschicht eingebetteten Glaskugeln starr entlang der Normale zur Platinenfläche abgestützt ist, können anschließend Prozesse insbesondere zur elektrischen Anbindung der Sensoreinrichtung mittels Bondpads an deren Oberseite an die Platine erleichtert sein. Beispielsweise kann die Sensoreinrichtung mittels Draht-Bonden an die Sensoreinrichtung angeschlossen werden, indem ein oder mehrere dünne Drähte mit der Sensoreinrichtung und der Platine verbunden werden, um auf diese Weise eine elektrische Kontaktierung der Sensoreinrichtung mit der Platine zu erhalten. Das Anschließen kann beispielsweise unter Verwendung eines Ultraschall-Draht-Bond-Verfahrens vorgenommen werden, indem Anschlussdrähte mittels Ultraschallschweißen mit der Sensoreinrichtung und der Platine verbunden werden. Der starre Aufbau entlang der Normale zur Platinenfläche gewährleistet dabei eine optimale Kraftübertragung.

Der der Erfindung zugrunde liegende Gedanke soll nachfolgend anhand der in den Figuren dargestellten Ausführungsbeispiele näher erläutert werden. Es zeigen:
- Fig. 1: eine schematische Ansicht einer Platinenanordnung, mit einer an einer Platine angeordneten Sensoreinrichtung;
- Fig. 2: eine schematische Ansicht beim Fügen der Sensoreinrichtung mit der Platine;
- Fig. 3: eine Ansicht der Platinenanordnung beim Fügen der Sensoreinrichtung;
- Fig. 4: eine schematische Ansicht eines Siebens von Glaskugeln zur Verwendung mit einer Klebstoffmasse zum Fügen der Sensoreinrichtung; und
- Fig. 5: eine schematische Ansicht eines implantierbaren medizinischen Geräts in einem menschlichen Körper.

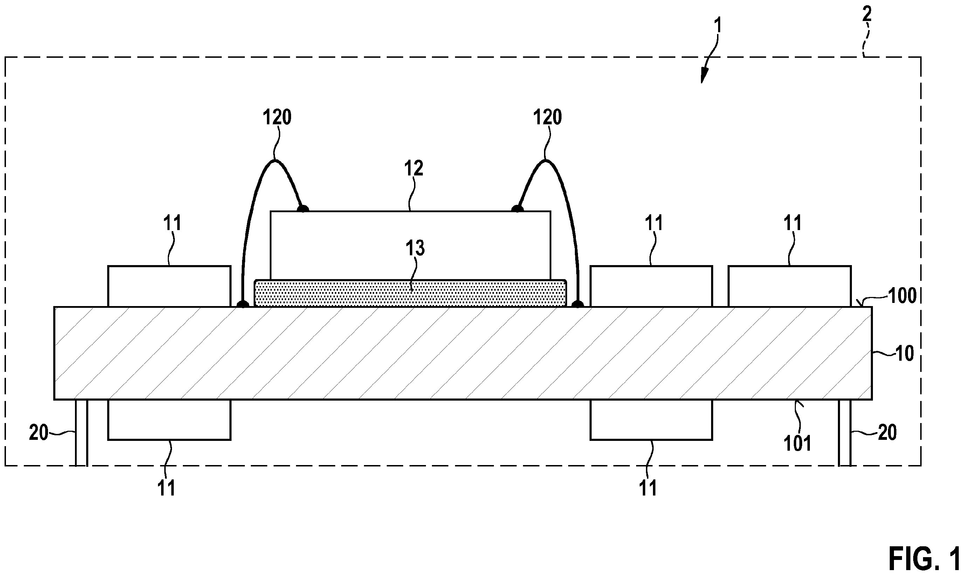

Eine in Fig. 1 schematisch dargestellte Platinenanordnung 1 ist Bestandteil eines medizinischen Geräts, beispielsweise eines implantierbaren Drucksensors, und ist in ein Gehäuse 2 des implantierbaren medizinischen Geräts eingefasst.

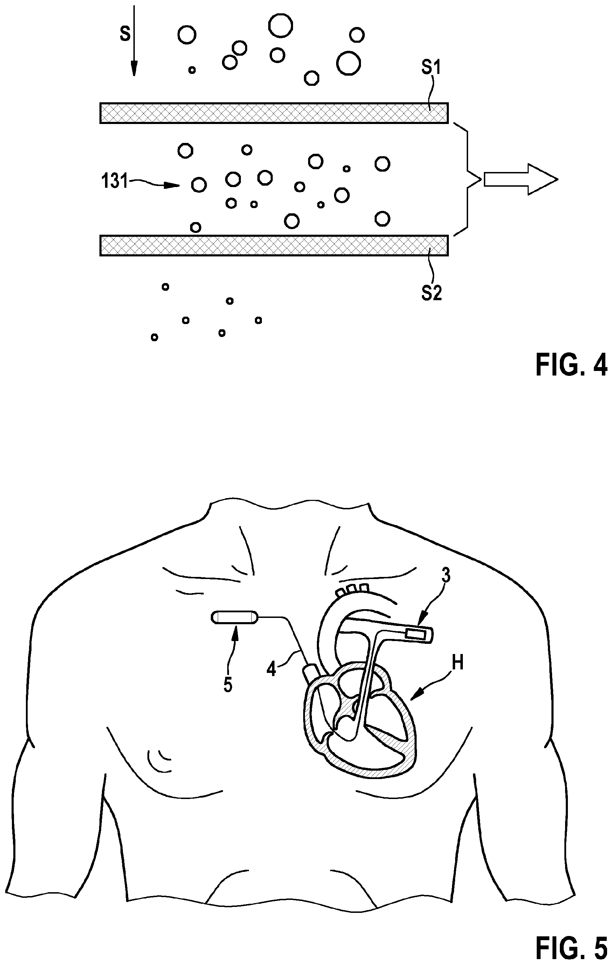

Wie aus Fig. 5 ersichtlich, kann ein solches implantierbares Gerät in Form eines implantierbaren Drucksensors 3 beispielsweise in ein Gefäß im Bereich des Herzens H eines Patienten implantiert sein, um zum Beispiel einen Blutdruck im Bereich des Herzens zum Zwecke eines Monitoring zu messen. Der Drucksensor 3 ist über eine Leitung 4 mit einer ebenfalls implantierten Steuereinrichtung 5 verbunden, die von dem Drucksensor 3 erhaltene Sensorsignale beispielsweise auswertet und verarbeitet und zum Beispiel an eine übergeordnete, externe Einheit außerhalb des Patienten beispielsweise unter Verwendung von Telemetrie kommuniziert. Alternativ kann der Drucksensor 3 kabellos ausgeführt sein und die Sensorsignale drahtlos an ein außerhalb des Körpers befindliches Gerät (z.B. ein Smartphone oder ein Tablet) übertragen (nicht dargestellt).

Wie aus Fig. 1 ersichtlich, weist die Platinenanordnung 1 eine Platine 10 auf, die über Befestigungseinrichtungen 20 nach Art von Festlagern mit dem Gehäuse 2 des medizinischen Geräts verbunden ist. Die Platine 10 trägt elektrische und elektronische Komponenten 11, beispielsweise einen Prozessor, einen Energiespeicher in Form einer Batterie oder dergleichen, und/oder passive elektrische Komponenten, wobei solche elektrischen und elektronischen Komponenten 11 an einander abgewandten Seiten 100, 101 der Platine 10 angeordnet sein können.

An einer oberen Seite 100 der beiden Seiten 100, 101 der Platine 10 ist zudem eine Sensoreinrichtung 12 angeordnet, die über eine Klebeschicht 13 mit der Platine 10 verbunden ist. Über zum Beispiel mittels Draht-Bonden angeschlossene Anschlussdrähte 120 ist die Sensoreinrichtung 12 mit der Platine 10, insbesondere mit an der Platine 10 gebildeten Leiterbahnen, verbunden, sodass die Sensoreinrichtung 12 auch elektrisch an die Platine 10 angebunden ist.

Im Betrieb können mechanische Spannungen über die Befestigungseinrichtungen 20 von dem Gehäuse 2 auf die Platine 10 übertragen werden. Um zu verhindern, dass solche mechanischen Spannungen von der Platine 10 auch in die Sensoreinrichtung 12 eingeleitet und gegebenenfalls zu einer mechanischen Verformung an der Sensoreinrichtung 12 führen (was ansonsten zu einer Verfälschung von über die Sensoreinrichtung 12 aufgenommenen Sensorsignalen führen könnte), hat die Klebeschicht 13 - über ihre Funktion der stoffschlüssigen Verbindung der Sensoreinrichtung 12 mit der Platine 10 hinaus - auch die Funktion, die Sensoreinrichtung 12 derart von der Platine 10 mechanisch zu entkoppeln, das insbesondere parallel zur Ebene der Platine 10 wirkende mechanische Spannungen nicht unmittelbar auf die Sensoreinrichtung 12 übertragen werden können.

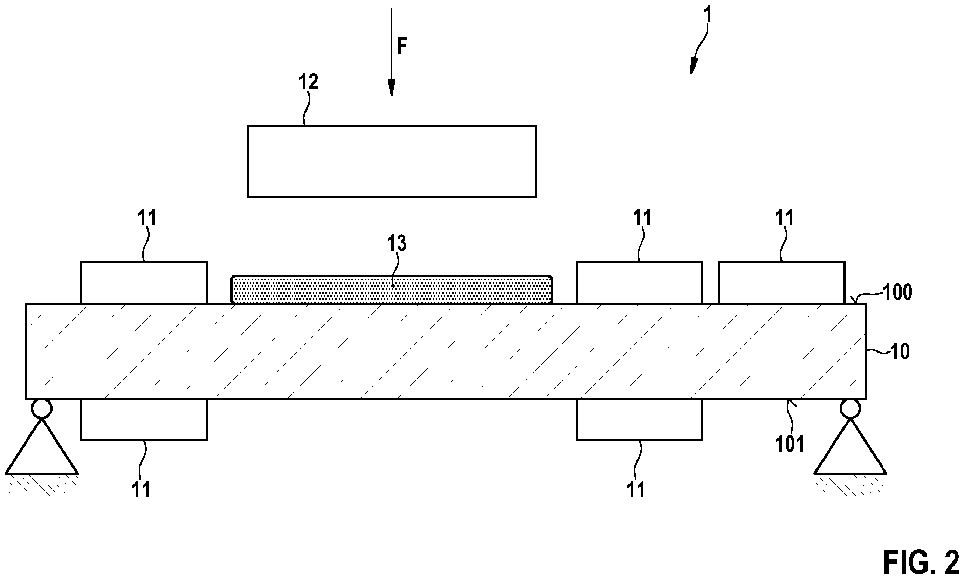

Wie schematisch in Fig. 2 dargestellt, wird die Sensoreinrichtung 12 zum Verbinden mit der Platine 10 entlang einer Fügerichtung F - die senkrecht zur Ebene der Platine 10 gerichtet ist - an die Platine 10 angesetzt und über die zwischen der Sensoreinrichtung 12 und der Platine 10 gebildete Klebeschicht 13 stoffschlüssig mit der Platine 10 verbunden. Um eine hinreichende mechanische Entkopplung der Sensoreinrichtung 12 von der Platine 10 zu erhalten, ist hierbei vorteilhaft, die Klebeschicht 13 hinreichend dick zu erhalten, sodass über die Klebeschicht 13 Verspannungen ausgeglichen und nicht unmittelbar von der Platine 10 in die Sensoreinrichtung 12 eingeleitet werden.

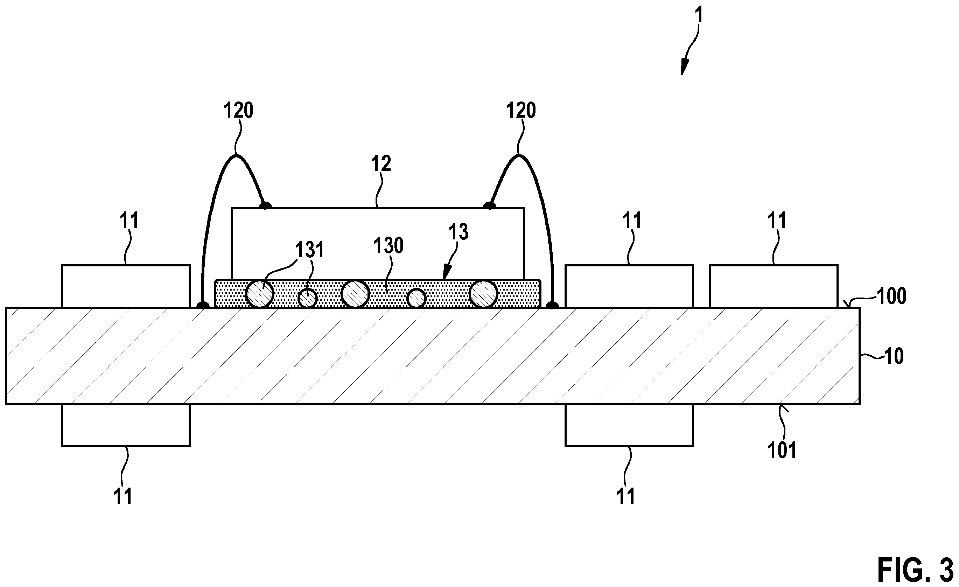

Zu diesem Zweck ist die Klebeschicht 13 bei dem in Fig. 3 dargestellten Ausführungsbeispiel durch eine Klebstoffmasse 130 gebildet, in die Glaskugeln 131 mit einem Durchmesser in einem definierten Bereich eingebettet sind. Bei der Klebstoffmasse 130 kann es sich beispielsweise um einen Silikonkleber, zum Beispiel einen 2-Komponenten-Kleber, handeln, der vor dem Aufbringen auf die Platine 10 mit den Glaskugeln 131 vermengt wird, sodass eine Zusammensetzung bestehend aus der Klebstoffmasse 130 und den Glaskugeln 131 erhalten und zur Ausbildung der Klebeschicht 13 vor Fügen der Sensoreinrichtung 12 auf die Platine 10 aufgebracht wird.

Die Glaskugeln 131 weisen vorzugsweise einen Durchmesser in einem Bereich zwischen 20 µm und 100 µm, beispielsweise zwischen 25 µm und 80 µm, vorzugsweise zwischen 30 µm und 50 µm, auf. Glaskugeln 131 können hierzu unter Verwendung von Sieben S1, S2, wie schematisch in Fig. 4 dargestellt, so gesiebt werden, dass ein Anteil von Glaskugeln 131 mit einem Durchmesser in einem gewünschten, definierten Bereich erhalten wird. Dies kann beispielsweise dadurch erfolgen, dass ein erstes Sieb S1 (betrachtet entlang einer Sieberichtung S) eine Maschenweite entsprechend der oberen Grenze des gewünschten Durchmesserbereichs und ein zweites Sieb S2, das dem ersten Sieb S1 in die Sieberichtung S nachgeordnet ist, eine Maschenweite entsprechend der unteren Grenze des Durchmesserbereichs aufweist. Glaskugeln 131, die das erste Sieb S1 passieren, aber an dem zweiten Sieb S2 aufgefangen werden, weisen einen Durchmesser in dem gewünschten Durchmesserbereich auf.

Solche Glaskugeln 131 weisen einen Elastizitätsmodul zwischen 40.000 N/mm² und 90.000 N/mm² auf und sind somit vergleichsweise starr. Über die Glaskugeln 131 wird die Sensoreinrichtung 12 somit entlang der Normalenrichtung, also senkrecht zur Platine 10, in vergleichsweise starrer Weise gegenüber der Platine 10 abgestützt.

Die Klebstoffmasse 130, bestehend aus einem Silikonkleber, ist demgegenüber vergleichsweise weich und elastisch, sodass insbesondere in Raumrichtungen parallel zur Ebene der Platine 10, die Sensoreinrichtung 12 von der Platine 10 in elastischer Weise entkoppelt ist. Horizontal zur Platine 10 gerichtete mechanische Spannungskräfte können somit nicht (unmittelbar) von der Platine 10 auf die Sensoreinrichtung 12 übertragen werden, sondern werden gedämpft und verfälschen damit Sensorsignale nicht.

Die Abstützung der Sensoreinrichtung 12 über die Glaskugeln 131 gegenüber der Platine 10 entlang der Normalenrichtung der Platine 10 hat den weiteren Vorteil, dass nach dem Fügen der Sensoreinrichtung 12 mit der Platine 10 vorzunehmende Prozessschritte mit verbesserter Prozesssicherheit durchgeführt werden können. So können Anschlussdrähte 120 mittels Draht-Bonden, zum Beispiel mittels Ultraschall-Draht-Bonden, mit der Sensoreinrichtung 12 und der Platine 10 verbunden werden, um die Sensoreinrichtung 12 elektrisch an die Platine 10 anzubinden. Mechanische Kräfte, die zum schweißenden Verbinden der Anschlussdrähte 120 mit der Sensoreinrichtung 12 in die Sensoreinrichtung 12 eingeleitet werden, werden mittels der (starren) Glaskugeln 131 übertragen.

Der der Erfindung zugrunde liegende Gedanke ist nicht auf die vorangehend geschilderten Ausführungsbeispiele beschränkt, sondern lässt sich auch in anderer Weise verwirklichen.

Grundsätzlich ist die Verwendung einer Klebeschicht, die aus einer Klebstoffmasse und darin eingebetteten Glaskugeln zusammengesetzt ist, nicht auf die Befestigung einer Sensoreinrichtung zum Wandeln eines Drucksignals in ein elektrisches Signal beschränkt, sondern auch andere Sensoreinrichtungen können über eine solche Klebeschicht mit einer Platine verbunden werden.

Eine Platinenanordnung der beschriebenen Art kann in ganz unterschiedliche implantierbare medizinische Geräte, zum Beispiel implantierbare Drucksensoren, Herzschrittmacher oder Defibrillatoren, integriert werden.

### Liste der Bezugszeichen

- 1: Platinenanordnung
- 10: Platine
- 100: Oberseite
- 101: Unterseite
- 11: Elektronische Komponente
- 12: Sensoreinrichtung (Transducer)
- 120: Anschlussdraht
- 13: Klebeschicht
- 130: Klebstoffmasse
- 131: Partikel (Glaskugeln)
- 2: Gehäuse
- 20: Befestigungseinrichtung
- 3: Implantierbarer Drucksensor
- 4: Anschlussleitung
- 5: Auswerteeinrichtung
- F: Fügerichtung
- H: Herz
- S: Sieberichtung
- S1, S2: Sieb

## Patentansprüche

1. Platinenanordnung (1) eines implantierbaren medizinischen Geräts (3), mit einer Platine (10) und einer an der Platine (10) angeordneten Sensoreinrichtung (12), die über eine Klebeschicht (13) mit der Platine (10) verbunden ist, wobei die Klebeschicht (13) aus einer Klebstoffmasse (130) gebildet ist, in die Glaskugeln (131) eingebettet sind, wobei die Glaskugeln (131) einen Elastizitätsmodul zwischen 40000 N/mm² und 90000 N/mm² aufweisen,
**dadurch gekennzeichnet dass** der Anteil der Glaskugeln (131) in der Klebeschicht (13) zwischen 5 Gew.% und 40 Gew.% beträgt.

2. Platinenanordnung (1) nach Anspruch 1, wobei die Glaskugeln (131) einen Durchmesser zwischen 20 µm und 100 µm, beispielsweise zwischen 25 µm und 80 µm, vorzugsweise zwischen 30 µm und 50 µm aufweisen.

3. Platinenanordnung (1) nach einem der Ansprüche 1 oder 2, wobei der Anteil der Glaskugeln (131) in der Klebeschicht (13) zwischen 8 Gew.% und 20 Gew.% beträgt.

4. Platinenanordnung (1) nach einem der vorangehenden Ansprüche, wobei die Klebstoffmasse (130) durch einen Silikonkleber gebildet ist.

5. Platinenanordnung (1) nach einem der vorangehenden Ansprüche, wobei die Sensoreinrichtung (12) als Transducer zum Wandeln eines Drucksignals in ein elektrisches Signal ausgebildet ist.

6. Implantierbares medizinisches Gerät (3) mit einer Platinenanordnung (1) nach einem der vorangehenden Ansprüche.

7. Verfahren zum Herstellen einer Platinenanordnung (1) eines implantierbaren medizinischen Geräts (3), bei dem eine Sensoreinrichtung (12) über eine Klebeschicht (13) mit einer Platine (10) verbunden wird, wobei die Klebeschicht (13) aus einer Klebstoffmasse (130) gebildet wird, in die Glaskugeln (131) eingemischt sind, wobei die Glaskugeln (131) einen Elastizitätsmodul zwischen 40000 N/mm² und 90000 N/mm² aufweisen,
**dadurch gekennzeichnet dass**
der Anteil der Glaskugeln (131) in der Klebeschicht (13) zwischen 5 Gew.% und 40 Gew % beträgt.

8. Verfahren nach Anspruch 7, wobei die Klebstoffmasse (130) aus zwei Komponenten eines Silikonklebers gemischt wird und nach dem Mischen der Klebstoffmasse (130) die Glaskugeln (131) in die Klebstoffmasse (130) eingemischt werden.

9. Verfahren nach Anspruch 7 oder 8, wobei nach dem Verbinden der Sensoreinrichtung (12) mit der Platine (10) Drahtanschlüsse (120) mittels Draht-Bonden an die Sensoreinrichtung (12) angeschlossen werden.

## Claims

1. A printed circuit board assembly (1) of an implantable medical device (3), comprising printed circuit board (10) and sensor device (12) disposed on the printed circuit board (10), the sensor device (12) is bonded to the circuit board (10) via an adhesive layer (13), wherein the adhesive layer (13) is formed from an adhesive compound (130) in which glass spheres (131) are embedded,
whereby
the glass spheres (131) have a modulus of elasticity between 40000 N/mm² and 90000 N/mm²
**characterized in that**
the proportion of glass spheres (131) in the adhesive layer (13) is between 5% and 40% by weight.

2. The printed circuit board assembly (1) according to claim 1, wherein the glass spheres (131) have a diameter between 20 µm and 100 µm, for example between 25 µm and 80 µm, preferably between 30 µm and 50 µm.

3. The printed circuit board assembly (1) according to one of claims 1 or 2, wherein the proportion of the glass spheres (131) in the adhesive layer (13) is between 8 wt.% and 20 wt.%.

4. The printed circuit board assembly (1) according to any one of the preceding claims, wherein the adhesive compound (130) is formed by a silicone adhesive.

5. The printed circuit board assembly (1) according to any one of the preceding claims, wherein the sensor device (12) is designed as a transducer for converting a pressure signal into an electrical signal.

6. An implantable medical device (3) comprising a printed circuit board assembly (1) according to any one of the preceding claims.

7. A method of manufacturing a printed circuit board assembly (1) of an implantable medical device (3), in which a sensor device (12) is bonded on the printed circuit board assembly (1) via an adhesive layer (13), wherein the adhesive layer (13) is formed from an adhesive compound (130) into which glass spheres (131) are mixed, wherein the glass beads (131) have a modulus of elasticity between 40000 N/mm² and 90000 N/mm²
**characterized in that**
the proportion of glass spheres (131) in the adhesive layer (13) is between 5 % by weight and 40 % by weight.

8. The method according to claim 7, wherein the adhesive compound (130) is composed of two components of a silicone adhesive is mixed and, after mixing the adhesive composition (130), the glass beads (131) are mixed into the adhesive compound (130).

9. The method of claim 7 or 8, wherein wire terminals (120) are connected to the sensor device (12) by means of wire bonding after bonding the sensor device (12) on the circuit board (10).

## Revendications

1. Agencement de platine (1) d'un appareil médical implantable (3), pourvu d'une platine (10) et d'un dispositif de capture (12) disposé sur la platine (10), qui est relié avec la platine (10) par le biais d'une couche adhésive (13), dans lequel la couche adhésive (13) est formée à base d'une masse d'adhésif (130) dans laquelle sont incorporées des billes de verre (131), dans lequel les billes de verre (131) présentent un module d'élasticité entre 40000 N/mm² et 90000 N/mm², **caractérisé en ce que** la proportion des billes de verre (131) dans la couche adhésive (13) se situe entre 5 % en poids et 40 % en poids.

2. Agencement de platine (1) selon la revendication 1, dans lequel les billes de verre (131) présentent un diamètre entre 20 µm et 100 µm, par exemple, entre 25 µm et 80 µm, de préférence entre 30 µm et 50 µm.

3. Agencement de platine (1) selon l'une des revendications 1 ou 2, dans lequel la proportion de billes de verre (131) dans la couche adhésive (13) se situe entre 8 % en poids et 20 % en poids.

4. Agencement de platine (1) selon l'une des revendications précédentes, dans lequel la masse d'adhésif (130) est formée par un adhésif silicone.

5. Agencement de platine (1) selon l'une des revendications précédentes, dans lequel le dispositif de capture (12) est conçu sous forme de transducteur permettant la conversion d'un signal de pression en un signal électrique.

6. Appareil médical implantable (3) pourvu d'un agencement de platine (1) selon l'une des revendications précédentes.

7. Procédé de fabrication d'un agencement de platine (1) d'un appareil médical implantable (3), chez lequel un dispositif de capture (12) est relié avec une platine (10) par le biais d'une couche adhésive (10), dans lequel la couche adhésive (13) est formée par une masse d'adhésif (130) dans laquelle sont incorporées des billes de verre (131), dans lequel les billes de verre (131) présentent un module d'élasticité entre 40000 N/mm² et 90000 N/mm²,
**caractérisé en ce que**
la proportion des billes de verre (131) dans la couche adhésive (13) se situe entre 5 % en poids et 40 % en poids.

8. Procédé selon la revendication 7, dans lequel la masse d'adhésif (130) est mélangée à partir de deux composés d'un adhésif silicone et les billes de verre (131) sont incorporées dans la masse d'adhésif (130) après le mélange de la masse d'adhésif (130).

9. Procédé selon la revendication 7 ou la revendication 8, dans lequel, après la liaison du dispositif de capture (12) avec la platine (10), des raccordements de câble (120) au moyen de liaisons filaires sont raccordés au dispositif de capture (12).
